# EUROPEAN PATENT APPLICATION

(11) **EP 0 774 268 A1**
(43) Date of publication of application: **21.05.1997**
(21) Application number: 96202112.7
(22) Date of filing: 25.07.1996
(51) Int. Cl.: A61M 5/50, A61M 5/32, A61M 5/315, A61M 5/24

(54) **A disposable syringe**

(30) Priority: 15.11.1995 IE 950874; 28.11.1995 IE 950900
(71) Applicant: Boyle, Francis, Balinfull, County Sligo (IE)
(72) Inventor: Boyle, Francis, Balinfull, County Sligo (IE)
(74) Representative: Boyce, Conor

(57) **Abstract**

A disposable syringe has an outer sleeve (12) and an inner sleeve (10) slidable within the outer sleeve for accommodating a vial (22) of fluid to be dispensed. The inner sleeve has a needle (14) at its front end which projects in use from the front end of the outer sleeve. A plunger (24) is insertable into the rear of the outer sleeve (12) and slidable forwardly of the outer sleeve for causing fluid in the vial to be expelled through the needle. The plunger and inner sleeve have cooperating means (38, 42) which cause the plunger and the inner sleeve to latch together so that upon withdrawal of the plunger the needle is automatically withdrawn into the outer sleeve.

## Description

This invention relates to a disposable syringe for a pre-filled vial.

According to the invention a disposable syringe comprises an outer sleeve, an inner sleeve for receiving a tubular vial of fluid to be dispensed, the inner sleeve having a hollow needle fixedly passing through its front end and being slidably accommodated in the outer sleeve for movement from an extended position in which the needle projects from the front end of the outer sleeve to a retracted position in which the needle is withdrawn into the outer sleeve, and a plunger insertable into the rear end of the outer sleeve and slidable forwardly of the outer sleeve for causing fluid in the vial to be expelled through the needle, the plunger and inner sleeve having cooperating means which cause the plunger and the inner sleeve to latch together so that upon rearward withdrawal of the plunger the inner sleeve is automatically withdrawn to its retracted position.

In the context of the present specification the end of the syringe from which the needle projects in use is deemed to be front of the syringe.

Embodiments of the invention will now be described, by way of example, with reference to the accompanying drawings, in which:
Fig. 1 is a sectional side view of a disposable syringe according to a first embodiment of the invention,
Fig. 2 is a sectional side view of the outer sleeve of the syringe of Fig. 1,
Fig. 3 is a sectional side view of the inner sleeve of the syringe of Fig. 1,
Fig. 4 is a side view of the plunger of the syringe of Fig. 1,
Fig. 5 is a perspective view of the plunger of Fig. 4,
Fig. 6 is a side view, partially in section, of a vial containing fluid for use in the syringe of Fig. 1,
Figs. 7 to 10 are views of the syringe of Fig. 1 at successive stages of use,
Fig. 11 is a sectional side view of a disposable syringe according to a second embodiment of the invention,
Fig. 12 is a sectional side view of the outer sleeve of the syringe of Fig. 11,
Fig. 13 is a sectional side view of the inner sleeve of the syringe of Fig. 11,
Fig. 14 is a side view of the plunger of the syringe of Fig. 11,
Fig. 15 is a side view, partially in section, of a vial containing fluid for use in the syringe of Fig. 11,
Figs. 16 to 19 are views of the syringe of Fig. 1 at successive stages of use, and
Figs. 20(a) and 20(b) are cross-sectional views illustrating the interconnection of the inner sleeve, outer sleeve and plunger of the syringe of Fig. 11.

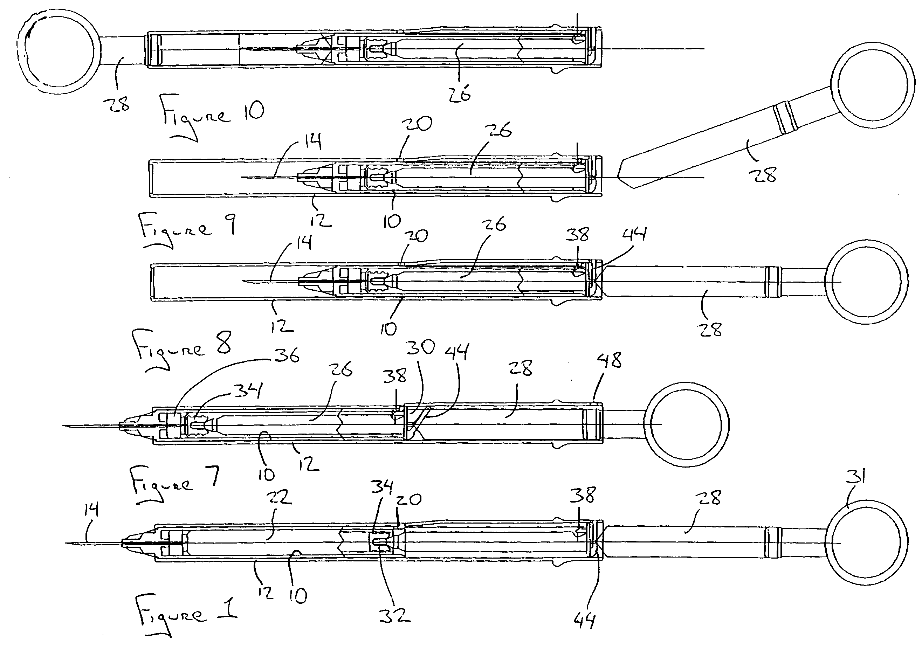

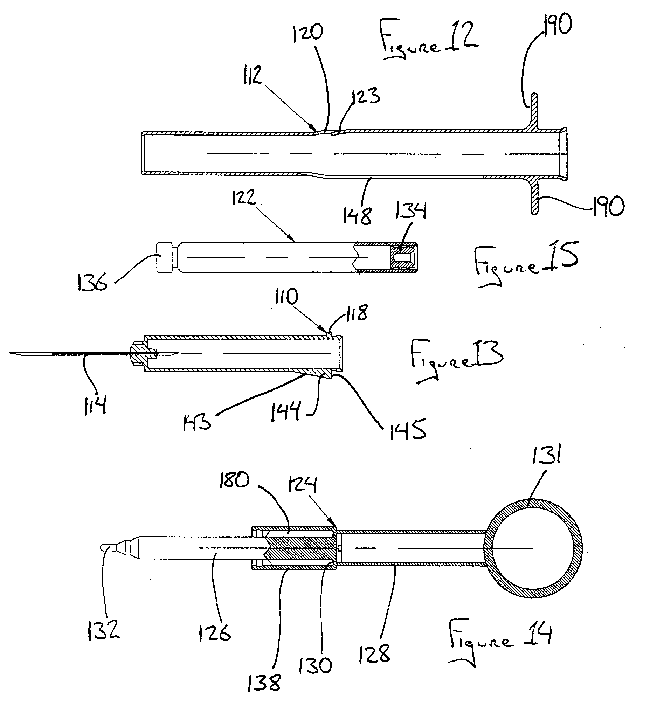

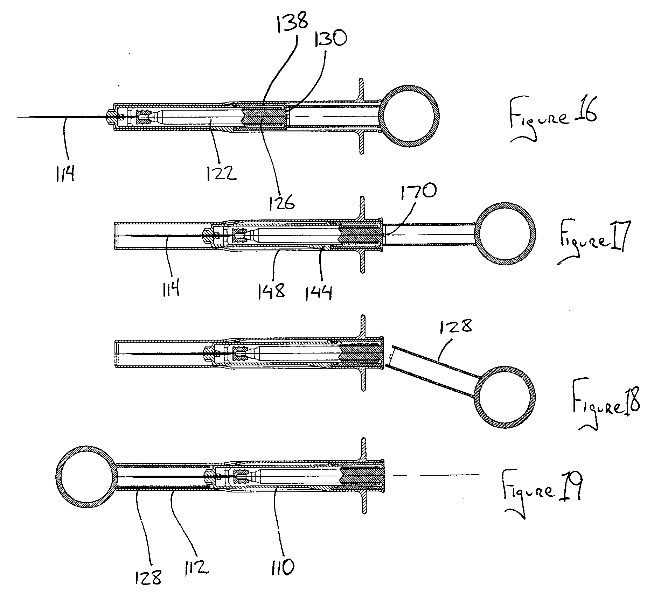

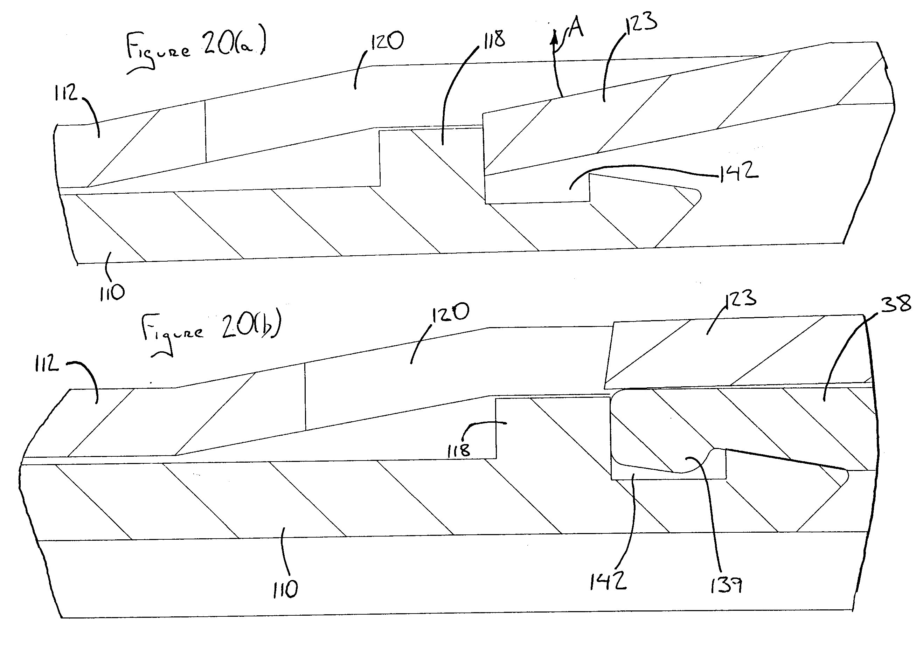

Referring now to Figs. 1 to 10 of the drawings, a first embodiment of disposable syringe comprises inner and outer substantially cylindrical sleeves 10 and 12 respectively, the inner sleeve 10 being accommodated within, and slidable coaxially of, the outer sleeve 12. The inner sleeve 10 has a hollow surgical steel needle 14, pointed at both ends, fixedly passing through its front end 16. The inner sleeve 10 may be slid relative to the outer sleeve 12 from an extended position in which the needle 14 projects from the front end of the outer sleeve 12 (Figs. 1 and 7) to a retracted position in which the needle 14 is withdrawn into the outer sleeve 12 (Figs. 8, 9 and 10).

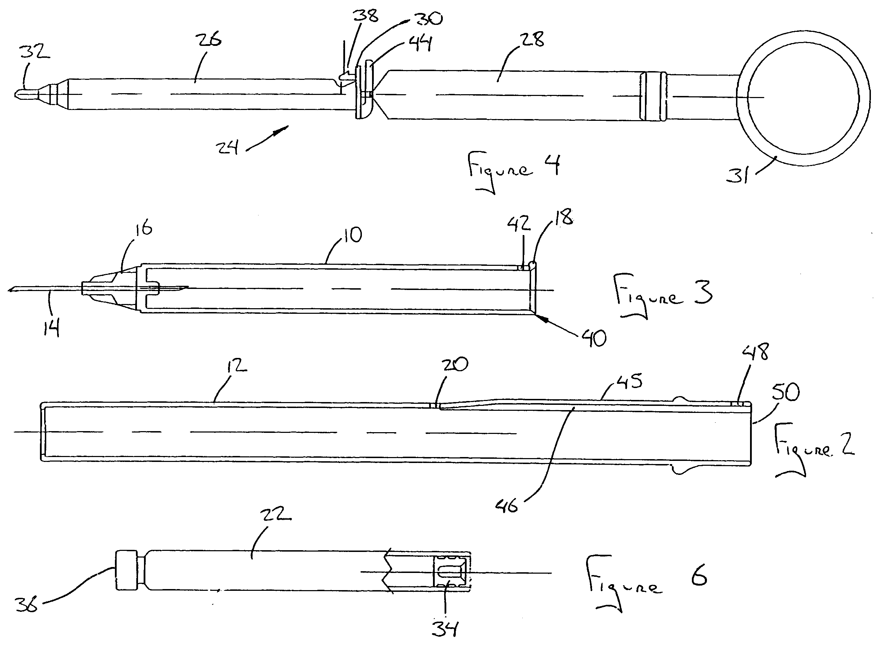

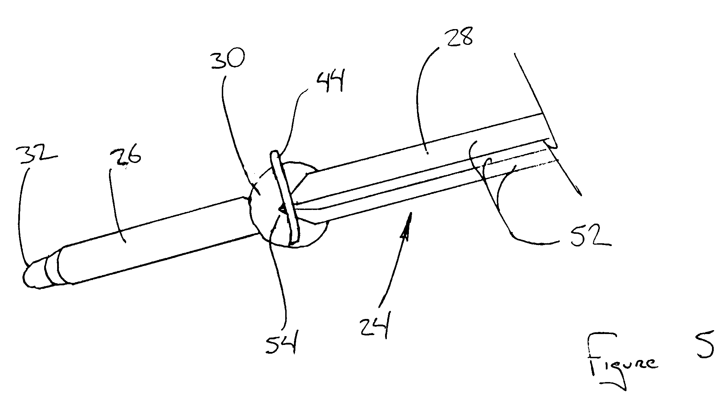

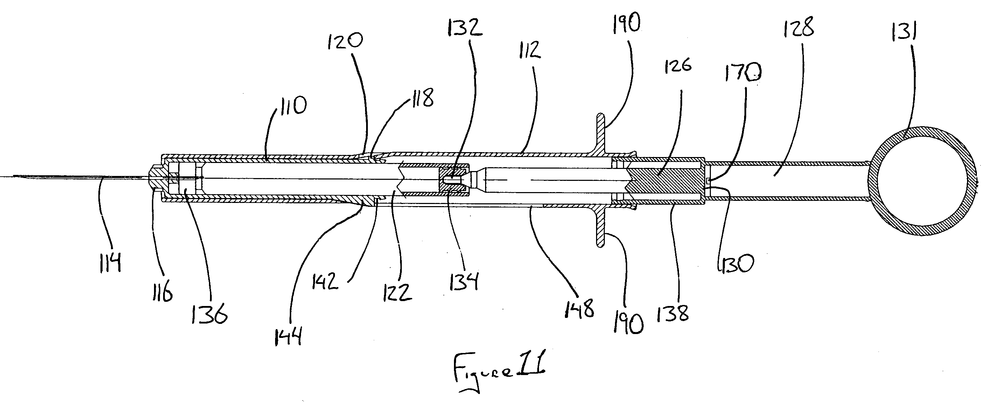

Prior to use of the syringe the inner sleeve 10 is held in place in the extended position by a small rounded projection 18 (Fig. 3) at the rear of the inner sleeve 10 which engages in a hole 20 (Fig. 2) in the outer sleeve 12. The exposed needle 14 is covered by a cap (not shown). The inner sleeve 10 is dimensioned to receive a conventional tubular vial 22 (Fig. 6) containing fluid to be dispensed. In this embodiment the inner and outer sleeves 10 and 12 are moulded from plastics material.

The syringe also includes a plunger 24 (Figs. 4 and 5) which can be inserted into the rear end of the outer sleeve 12, as seen in Fig. 1. The plunger comprises front and rear parts 26 and 28 respectively, separated by a disc 30, and a thumb hole 31. The front part 26 is of cylindrical cross-section dimensioned to slide within the vial 22 while the rear part 28 is of cruciform cross-section dimensioned, like the disc 30, to slide within the outer sleeve 12. In this embodiment the parts 26 and 28 of the plunger together with the disc 30 are formed as an integral moulding of plastics material.

When inserted into the rear end of the outer sleeve 12 the front end 32 of the plunger 24 engages in a rubber piston 34 at the rear of the vial 22. Then, when the plunger 24 is squeezed into the outer sleeve 12 (the outer sleeve has finger holds, not shown, which provide the necessary purchase for the fingers), the plunger first pushes the vial 22 onto the part of the needle 14 within the inner sleeve 10 so that the needle punctures the front end 36 of the vial and enters the vial, whereafter further forward movement of the plunger 24 causes the front part 26 to slide within the vial 22, pushing the piston 34 ahead of it to expel the fluid in the vial from the needle 14. Fig. 7 shows the syringe after the plunger has completed the forward stroke, and substantially all the fluid has been expelled from the vial.

The front surface of the disc 30 has a forwardly extending hook 38. Towards the end of the forward stroke of the plunger 24 the hook 38 engages and is resiliently deflected inside the rear open end 40 of the inner sleeve 10. Finally, at the end of the forward stroke (Fig. 7) the hook 38 engages in a hole 42 in the inner sleeve 10. Now the plunger 24 and the inner sleeve 10 are positively latched together, so that when the plunger 24 is withdrawn the essentially frictional force of engagement between the projection 18 and hole 20 is overcome and the inner sleeve is automatically withdrawn to its retracted position (Fig. 8). This causes the needle 14 to be retracted within the outer sleeve 12 and thus mitigates the danger of stick injuries after use of the syringe.

To ensure that the hook 38 is properly aligned to engage the hole 42 at the end of the forward stroke it is necessary to ensure that the plunger 24 does not rotate relative to the outer sleeve 12 during the forward stroke (it will be appreciated that the engagement of the projection 18 in the hole 20 already maintains the inner sleeve 10 against rotation in the outer sleeve 12). To this end the rear surface of the disc 30 has a resilient finger 44 which in its unstressed state extends beyond the edge of the disc, and the outer sleeve 12 has widened portion 45 defining a narrow internal longitudinal channel 46 with a hole 48 at its rear end.

When the plunger 24 is first inserted in the outer sleeve 12 the finger 44 is resiliently deflected rearwardly inside the rear end 50 of the channel 46 but soon reaches the hole 48 whereupon the free end of the finger 44 is able to relax into the hole 48 to maintain the plunger 24 at that initial position if desired. Then, when a forward stroke of the plunger 24 is made, the finger 44 is deflected rearwardly out of the hole 48 and into the channel 46 once again, and slides along the channel 46 to prevent the plunger 24 rotating in the outer sleeve 12. The rearwardly deflected position of the finger 44 is shown in Fig. 7.

When the plunger 24 is withdrawn the finger 44 slides back up the channel 46 and ultimately re-engages the hole 48. However, in this direction of movement the finger 44 cannot move past the hole 48, because the presence of the disc 30 immediately in front of the finger 44 prevents the latter from deflecting far enough in the forward direction to clear the hole 48. Thus, as well as guiding the movement of the plunger 24 within the outer sleeve 12, the finger 44 also prevents the plunger 24 from being extracted too far from the outer sleeve 12. In effect the finger 44 and the hole 48 constitute a one-way stop mechanism which allows the plunger 24 to be slid beyond the Fig. 1 position in the forward direction of movement but not in the opposite direction of movement.

The four flights or webs 52 of the rear part 28 of the plunger 24 taper to a narrow junction 54 with the disc 30. Thus, when the plunger 24 is withdrawn to the Fig. 8 position the rear part 28 may be snapped off, Fig. 9, and inserted into the front end of the outer sleeve 12. This prevents the syringe accidentally being used again, and also prevents infection spreading from the needle 14.

Referring now to Figs. 11 to 20 of the drawings, a second embodiment of disposable syringe comprises inner and outer substantially cylindrical sleeves 110 and 112 respectively, the inner sleeve 110 being accommodated within, and slidable coaxially of, the outer sleeve 112. The inner sleeve 110 has a hollow surgical steel needle 114, pointed at both ends, fixedly passing through its front end 116. The inner sleeve 110 may be slid relative to the outer sleeve 112 from an extended position in which the needle 114 projects from the front end of the outer sleeve 112 (Figs. 11 and 16) to a retracted position in which the needle 114 is withdrawn into the outer sleeve 112 (Figs. 17, 18 and 19).

Prior to use of the syringe the exposed needle 114 is covered by a cap (not shown). The inner sleeve 110 has a small projection 118, Fig. 20(a), disposed towards the rear of the inner sleeve 110, in the vicinity of a hole 120 in the outer sleeve 112. A resilient finger 123 is formed in the outer sleeve 112 and in its unstressed position, Fig. 20(a), the forward end of the finger 123 extends inwardly of the hole 120 to form a stop member against which the projection 118 bears to prevent the inner sleeve 110 from being pushed into the retracted position.

The inner sleeve 110 is dimensioned to receive a conventional tubular vial 122 (Fig. 15) containing fluid to be dispensed. In this embodiment the inner and outer sleeves 110 and 112 are moulded from plastics material.

The syringe also includes a plunger 124 (Fig. 14) which can be inserted into the rear end of the outer sleeve 112, as seen in Fig. 11. The plunger comprises front and rear parts 126 and 128 respectively, separated by a circular plate 130, and a thumb hole 131. The front part 126 is of cylindrical cross-section dimensioned to slide within the vial 122, the rear part 128 is of a larger cylindrical cross-section dimensioned to fit with small annular clearance within the outer sleeve 112, and the plate 130 is of larger diameter again and is dimensioned to slide within the outer sleeve 112. In this embodiment the parts 126 and 128 of the plunger together with the plate 130 are formed as an integral moulding of plastics material.

When inserted into the rear end of the outer sleeve 112 of the front end 132 of the plunger 124 engages in a rubber piston 134 at the rear of the vial 122. Then, when the plunger 124 is squeezed into the outer sleeve 112 (the outer sleeve has finger holds 190 which provide the necessary purchase for the fingers), the plunger first pushes the vial 122 onto the part of the needle 114 within the inner sleeve 110 so that the needle punctures the front end 136 of the vial and enters the vial, whereafter further forward movement of the plunger 124 causes the front part 126 to slide within the vial 122, pushing the piston 134 ahead of it to expel the fluid in the vial from the needle 114. Fig. 16 shows the syringe after the plunger has completed the forward stroke, and substantially all the fluid has been expelled from the vial.

A cylindrical actuating sleeve 138 projects forwardly from the front surface of the plate 130. The sleeve 138 has an outer diameter equal to the diameter of the plate 130 and slides within the outer sleeve 112. An annular gap 180 is defined between the actuating sleeve 138 and the smaller diameter front part 126, Fig. 14. Towards the end of the forward stroke of the plunger 124, the cylindrical wall of the vial 122 slides into the gap 180 and the front end of the actuating sleeve 138 engages and resiliently deflects the finger 123 outwards in the direction of the arrow A, Fig. 20(a), so as to clear the projection 118. Finally, at the end of the forward stroke, Fig. 20(b), the forward end of the sleeve 138 slides over the rear end of the inner sleeve 110 so that a shallow radially inward projection 139 on the forward end of the sleeve 138 snaps into engagement with a peripheral groove 142 at the rear end of the inner sleeve 110. Now the plunger 124 and the inner sleeve 110 are positively latched together, and the finger 123 is clear of the inner sleeve 110, so that when the plunger 124 is withdrawn the inner sleeve is automatically withdrawn to its retracted position (Fig. 17). This causes the needle 114 to be retracted within the outer sleeve 112 and thus mitigates the danger of stick injuries after use of the syringe.

An outwardly projecting fin 144 is formed on the side of the inner sleeve 110 opposite the projection 118, Fig. 11, and has a forwardly sloped profile 143 terminating in a stepped rear end 145. When the syringe is assembled, the inner sleeve 110 is inserted in the outer sleeve 112 and the sloped profile 143 of the fin 144, acting like a cam, momentarily deforms the outer sleeve 112 slightly so as to permit the fin 144 to locate in a longitudinal channel 148 running along a portion of the outer sleeve 112. When the plunger 124 is withdrawn the fin 144 slides back up the channel 148 until the rear end 145 of the fin 144 engages the rear end of the channel 148, Fig. 17. Thus, the fin 144 prevents the plunger 124 from being extracted too far from the outer sleeve 112. In effect the fin 144 and the channel 148 constitute a one-way stop mechanism which allows the plunger 124 to be slid beyond the Fig. 11 position in the forward direction of movement but not in the opposite direction of movement.

A plurality of portions 170 are removed from the rear part 128 of the plunger 124 in the region of the plate 130, thus weakening the plunger. Thus, when the plunger 124 is withdrawn to the Fig. 17 position the rear part 128 may be snapped off, Fig. 18, and inserted into the front end of the outer sleeve 112, Fig. 19. This prevents the syringe being used again accidentally, and also prevents infection spreading from the needle 114.

## Claims

1. A disposable syringe comprising an outer sleeve (12), an inner sleeve (10) for receiving a tubular vial (22) of fluid to be dispensed, the inner sleeve (10) having a hollow needle (14) fixedly passing through its front end and being slidably accommodated in the outer sleeve (12) for movement from an extended position in which the needle (14) projects from the front end of the outer sleeve (12) to a retracted position in which the needle (14) is withdrawn into the outer sleeve (12), and a plunger (24) insertable into the rear end of the outer sleeve (12) and slidable forwardly of the outer sleeve (12) for causing fluid in the vial (22) to be expelled through the needle (14), the plunger (24) and inner sleeve (10) having cooperating means (38, 42) which cause the plunger (24) and the inner sleeve (10) to latch together so that upon rearward withdrawal of the plunger (24) the inner sleeve (10) is automatically withdrawn to its retracted position.

2. A disposable syringe as claimed in claim 1, wherein the plunger (24) has means at its front end (32) for engagement with a piston (34) at the rear end of the vial (22), forward movement of the plunger (24) causing the needle (14) to puncture the vial (22) at its front end and fluid to be expelled from the needle (14) by forward movement of the piston (34) in the vial (22).

3. A disposable syringe as claimed in claim 1 or 2, wherein the outer sleeve (12) and one of the plunger (24) and inner sleeve (10) have second cooperating means (44, 48 or 144, 148) which engage to prevent withdrawal of the inner sleeve (10) beyond a predetermined point.

4. A disposable syringe as claimed in claim 3, wherein the second cooperating means comprises an aperture (48) in the sidewall of the outer sleeve (12) and a resilient finger (44) on the plunger (24) for engaging the aperture (48) when the plunger (24) is at the said predetermined point, the finger (44) being deflectable out of the aperture (48) in the forward direction of movement of the plunger (24) but resisting such deflection in the opposite direction.

5. A disposable syringe as claimed in claim 4, wherein the sidewall of the outer sleeve (12) has a longitudinal channel (46) which is engaged by the finger (44) after its deflection out of the aperture (48) so as to substantially prevent rotation of the inner sleeve (10) relative to the outer sleeve (12) during forward movement of the plunger (24).

6. A disposable syringe as claimed in claim 3, wherein the second cooperating means comprises a longitudinal channel (148) in the outer sleeve (112) which is slidably engaged by a projection (144) on the inner sleeve (110), the projection (144) being so shaped as to permit the inner sleeve (110) to be inserted into the rear end of the outer sleeve (112) such that the projection (144) locates in the channel (148) but preventing withdrawal of the inner sleeve (10) beyond the point at which the projection (144) abuts the rear end of the channel (148).

7. A disposable syringe as claimed in any one of claims 3 to 6, wherein the plunger (24) is formed with front and rear parts (26, 28) with the rear part (28) being detachable from the front part (26), the rear part (28) projecting from the rear of the outer sleeve (12) when the plunger (24) is at the said predetermined point.

8. A disposable syringe as claimed in any preceding claim, wherein the first cooperating means comprises a hook (38) on the plunger (24) which engages an aperture (42) in the sidewall of the inner sleeve (10) at the end of the forward movement of the plunger (24).

9. A disposable syringe as claimed in any preceding claim, wherein the first cooperating means comprises a projection (139) on the plunger (124) which engages a groove (142) in the sidewall of the inner sleeve (110) at the end of the forward movement of the plunger (124).

10. A disposable syringe as claimed in any preceding claim, wherein the plunger (24) and the outer sleeve (12) have third cooperating means (18, 20 or 118, 123) for releasably holding the inner sleeve (10) in the extended position in the outer sleeve (12) prior to use of the syringe.

11. A disposable syringe as claimed in claim 10, wherein the third cooperating means comprises a projection (18) on one of the inner sleeve (10) and outer sleeve (12) which engages an aperture (20) in the other of the inner sleeve and outer sleeve, the holding action of the third cooperating means being overcome by rearward withdrawal of the plunger (24) to permit the inner sleeve (10) to be withdrawn to the retracted position.

12. A disposable syringe as claimed in claim 10, wherein the third cooperating means comprises a member (123) on the outer sleeve (112) which extends resiliently into engagement with the rear end of the inner sleeve (110) to hold the latter in the extended position, and means (138) on the plunger (124) for deflecting the resilient member (123) outwardly clear of the inner sleeve (110) to permit the latter to be withdrawn by the plunger (124).
